# EUROPEAN PATENT APPLICATION

(11) **EP 3 889 580 A1**
(43) Date of publication of application: **06.10.2021**
(21) Application number: 20020155.6
(22) Date of filing: 05.04.2020
(51) Int. Cl.: G01N 21/3577, G01N 21/45

(54) **SYSTEM AND METHOD OF MEASURING CONTAMINANTS IN A SUBSTANTIALLY TRANSLUCENT MATERIAL, SUCH AS WATER**

(71) Applicant: TGTW Group B.V., 2909 LA Capelle aan den Ijssel (NL)
(72) Inventor: Kleczewski, Lazlo, 1511 WV Oostzaan (NL)
(74) Representative: Kosti, Vasiliki

(57) **Abstract**

A system for sensing analyte in at least partly translucent material, comprising one or more radiation sources configured for successively providing radiation at a first and a second wavelength, respectively, two or more waveguides for simultaneously transmitting the radiation at each wavelength provided by the radiation source, a first waveguide being a reference waveguide and a second being a sensing waveguide; and measuring means for measuring a phase difference between the radiation waves from the reference waveguide and the measuring waveguide, resp.. The present method can be used for measuring contaminants such as Fe, Sn, and/or Pb in oil related products, such as carburant or lubricant.

## Description

Interferometers (Mach-Zehnder, Michelson, Young etc.) are primarily used to measure phase differences (speed difference, refractive index) between two waves of light or other radiation for which purpose they show sufficient sensitivity even at the speed of light.

The present invention provides a system for sensing analyte in at least partly translucent material, comprising:
- one or more radiation sources configured for successively providing radiation at a first and a second wavelength, resp.;
- two or more waveguides for simultaneously transmitting the radiation at each wavelength provided by the radiation source, a first waveguide being a reference waveguide and a second being a sensing waveguide; and
- measuring means for measuring a phase difference between the radiation waves from the reference waveguide and the measuring waveguide, resp..

Preferably the different wavelength are provided by a number of monochromatic light sources which are switched ON/OFF one after the other.

The different wavelengths can preferably also be provided by a single (broad band) source, e.g. by using optical filtering and/or changing the temperature of the source.

The research of electrochemical reactions nowadays goes into the direction of measuring the dielectric properties of a medium as a function of frequency. It is an experimental method of characterizing electrochemical systems. This technique measures the impedance of a system over a range of frequencies. Often, data obtained by electrochemical impedance spectroscopy (EIS) are expressed graphically. The frequency ranges are each suited for different media. At high frequencies electronics may become a limiting factor.

The present invention also provides a method of measuring the quantity of analyte in at least partly translucent material, wherein the above system is used, and wherein both the absorption and the phase difference for different wavelengths one after the after is measured such that the composition and quantity of different analytes can be determined.

Preferably the radiation is in the high frequency optical range, viz. extending from near IR into UV, so that the measurements are very accurate for optically translucent materials, also called transparent materials.

The present preferred method can be used for measuring contaminants such as Fe, Sn, and/or Pb in oil related products, such as carburant or lubricant. Also the quality of pharmaceutical solutions and liquid food products, such as olive oil can de monitored. Preferably the method is used for monitoring contaminants in water; if phase difference values increase the operator can easily check the spectrum of the measured analytes.

Further details, advantages and characteristics of the present invention are clarified in the following description of preferred embodiments thereof referring to the annexed drawing, in which show:
Figure 1 a schematic diagram of a preferred embodiment of the present invention; and
Figure 2 a schematic diagram of another preferred embodiment of the present invention.

Substances showing transparent properties are most likely equal, if the refractive indexes are. Small changes to the material composition will typically result in subtle changes in the refractive index that can be measured.

Using an interferometer is a highly sensitive method that is able to detect very subtle changes in the refractive index.

For example water from different wells, will typically have slightly different refractive indexes that could both be perfectly safe for consumption. While a small trace amount of certain harmful chemicals dissolved in the water can cause similar changes in the refractive index, making it unsafe for consumption. It would be useful to give early warning of a possible problem, which allows for preventing harmful chemicals ending up in the drinking water supplied to users.

Once a change in the refractive index of the substance (water) would have been detected, additional testing could be done to identify the source of the change. This would imply taking a sample and analyse this sample in a lab.

Further sensors could also be used to search for contamination candidates.

This takes times also because each contaminant requires a dedicated sensor. A setup that is able to detect any contaminant is practically impossible.

A method that has both a high sensitivity and the ability to identify the chemicals is disclosed in the present patent application.

The refractive sensor, that is using monochromatic light, required for the internal interferometer, effectively gets two signals, the refractive index, and the light absorption at that particular wavelength (within a very narrow band of e.g 10 nm). The refractive measurement uses a single wavelength at a time.

According to the present patent disclosure a number a light source are used that generate different wavelengths in sequence, or a wavelength sweep, so that it becomes possible to get more detailed information of the exact wavelengths with the amount of light absorption, and thus also obtain information about the chemical composition of the material tested by the refractive sensor, such as in gas chromatography in which method the measured spectrum is used to identify the composition of materials.

The present patent disclosure allows the high sensitivity of the refractive index measurements, and thus detecting very small changes while at the same time identifying the chemical compounds that are the cause of the change in the refractive index, and thus bypassing the need to do time consuming external testing to evaluate if corrective measures are to be taken.

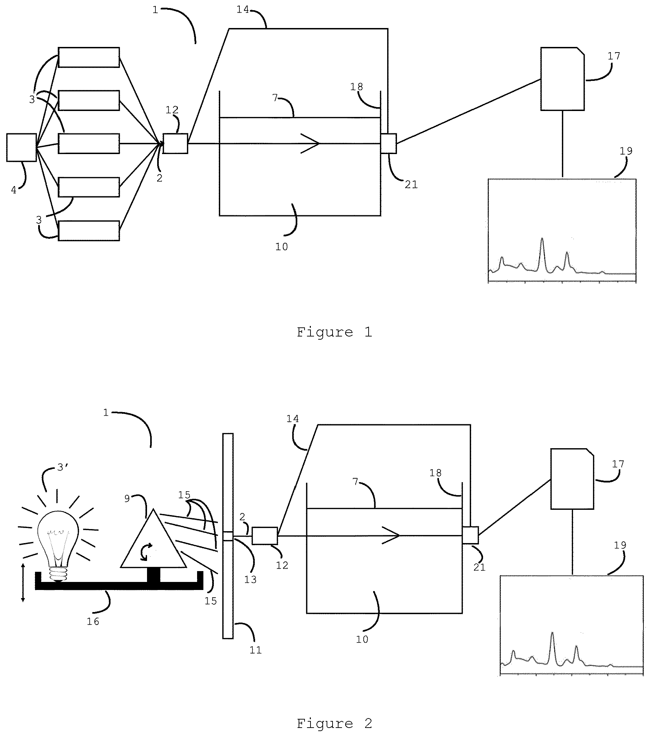

A light source that would make this possible could be for example in a system 1 (Fig. 1) a series of solid state lasers / LEDs 3 each tuned to a single wavelength and each connected to a voltage source 4. At 2 the lasers/LEDs are connected to a pair of waveguides, of which a sensing waveguide extends through a substance 10, such as water with some salts or other chemical or physical impurities dissolved or emulsified.

The sensing waveguide extending from connection point 2 under the fluid surface 7 in a holder 18 through an end wall thereof into measuring unit 21 is e.g. provided with a window through which the refractive index (i.e. the velocity of light) is influenced by the amount of analyte present in the fluid.

In measuring unit 21 the phase difference relative to the radiation in the reference waveguide is measured by a number of photo diodes which detect the interference pattern between the two waves as well as the absorption of the waves at the particular wavelength.

As schematically indicated at 19 a spectrum for analysing the composition of the analyte (contaminant) is provided as well as a very accurate measure 17 of the difference in refractive index related to the amount of the contaminant.

As soon as the phase difference start changing an operator can see whether also the spectrum starts changing.

The Laser/LEDS are switched on/off in sequence such that only one is active at a given moment in time. It would also be possible to use the wavelength tuning mechanism of these light sources to sweep through a wavelength range.

Another method (Fig. 2) uses a single white light source (for example a halogen lamp) 3' on a movable table 16, which light source shines through a rotating prism 9 to a slotted filter plate 11 showing opening 13 so that only a very narrow band of the light rays 2 from the prism is allowed to actually pass through. This will then create a light with the very narrow wavelength range that can be used by the interferometer of the refractive sensor.

By moving the light source, the prism or the slotted plate, or a combination thereof, it is possible to change the wavelength that is used by the sensor and thus sweeping through the wavelengths.

The reference numerals of Fig.1 are also used for the same or equivalent parts in Fig.2.

Other possibilities as not limiting examples, are the use of narrow band colour filters, and any other means that would allow the creation of a light with a very narrow wavelength band, whereby the base wavelength can be modified over a range.

The present invention is not limited to the description and embodiments above; the requested rights are determined by the following claims, within the scope of which many modifications are feasible.

## Claims

1. System for sensing analyte in at least partly translucent material, comprising:
- one or more radiation sources configured for successively providing radiation at a first and a second wavelength respectively;
- two or more waveguides for simultaneous transmitting the radiation at each wavelength provided by the radiation source, a first waveguides being a reference waveguide and a second being a sensing waveguide; and
- measuring means for measuring a phase difference between the radiation waves from the reference waveguide and the measuring waveguide resp.

2. System according to claim 1 wherein the radiation source provides different wavelengths, by a filter to transmit one wavelength after the other to the wave guides.

3. System according to claim 1 or 2 wherein the at least one radiation source comprises two or more radiation source each having a different wavelength relative to another.

4. System according to claims 1, 2 or 3, wherein the measuring means also include means for measuring absorption of the radiation absorbed in the measuring wave guide.

5. System according to any of claims 1-4, wherein the different wavelengths extend from the infrared spectrum into the ultraviolet range, preferably including the visual wave length.

6. System according to any of the claim 1-5, wherein the measuring means comprise an array of photodetectors to establish a phase difference between the waves from the measuring wave guide and the reference wave guide.

7. Method of measuring the quantity of analyte in at least partly translucent material, wherein a system accordingly to any of the claim 1-6 is used, and wherein both the absorption and the phase difference for different wave lengths one after the after is measured such that the composition and quantity of different analytes can be determined.

8. Method of claim 7, wherein the radiation source comprises a number of lasers/LEDs, preferably five to fifteen, to provide (near) visible light at different wavelength having a narrow bandwidth.

9. Method of claim 8, wherein each of the Lasers/LEDs is switched ON and OFF, subsequentially in time

10. Method of claim 7,8 or 9, wherein the absorption for different wavelength is measured as soon as it is sensed that the quantity of analyte has changed.

11. Method of any of any of claims 7-10, wherein the translucent material is water, and the analyte is salt or any other chemical or physical water contaminant.

12. Method according to claim 11, wherein it is established by the measuring means that the water is safe for consumption.

## Amended claims

### Amended claims in accordance with Rule 137(2) EPC.

1. Method of measuring the quantity of analyte in at least partly translucent material, wherein both the absorption and the phase difference for different wavelengths one after the after is measured such that the composition and quantity of different analytes can be determined, and wherein a system for sensing analyte in at least partly translucent material is used, said system comprising:
- one or more light radiation sources configured for successively providing, or sweeping, light radiation at a first and a second wavelength, respectively;
- two waveguides for simultaneous transmitting the radiation at each wavelength provided by the radiation source, a first waveguides being a reference waveguide and a second being a sensing waveguide; and
- measuring means for measuring a phase difference between the radiation waves from the reference waveguide and the measuring waveguide respectively.

2. Method of claim 1, wherein the one or more light radiation sources comprises a number of lasers/LEDs to provide (near) visible light at different wavelength having a narrow bandwidth, wherein each of the Lasers/LEDs is switched ON and OFF, subsequentially in time such that the light radiation is swept over different wavelengths.

3. Method of claim 2, wherein the number of lasers/LEDs between five and fifteen.

4. Method of claim 1,2 or 3, wherein the absorption for different wavelength is monitored when it is is sensed that the quantity of analyte has changed.

5. Method of any of claims 1-4, wherein the translucent material is water, and the analyte is salt or any other chemical or physical water contaminant.

6. Method according to claim 5, wherein it is established by the measuring means that the water is safe for consumption.

7. System configured to be usable in the method according to any of claims 1-6 wherein the light radiation source provides a range of different wavelengths, by a filter to transmit light radiation of one wavelength after the other to the wave guides.

8. System according to claim 7 wherein the at least one radiation source comprises two or more radiation source each having a different wavelength relative to one another.

9. System according to claims 7 or 8, wherein the measuring means also include means for measuring absorption of the radiation absorbed in the measuring wave guide.

10. System according to any of claims 7-9, wherein the different wave length extend from the infrared spectrum into the ultraviolet range, preferably including the visual wave length.

11. System according to any of the claim 7-10, wherein the measuring means comprise an array of photodetectors to establish a phase difference between the waves from the measuring wave guide and the reference wave guide.
